# EUROPEAN PATENT SPECIFICATION

(11) **EP 3 435 876 B1**
(45) Date of publication and mention of the grant of the patent: **23.06.2021**
(21) Application number: 17714714.7
(22) Date of filing: 30.03.2017
(51) Int. Cl.: A61B 8/08, A61B 8/12, A61B 8/00, A61B 5/00

(54) **TISSUE AND VASCULAR PATHWAY MAPPING UTILIZING PHOTOACOUSTIC AND ULTRASOUND TECHNIQUES**
GEWEBE- UND GEFÄSSBAHNKARTIERUNG MIT FOTOAKUSTISCHEN UND ULTRASCHALLTECHNIKEN
CARTOGRAPHIE DE LA VOIE TISSULAIRE ET VASCULAIRE À L'AIDE DE TECHNIQUES PHOTOACOUSTIQUES ET ULTRASONORES

(30) Priority: 30.03.2016 US 201662315117 P
(43) Date of publication of application: 06.02.2019
(73) Proprietor: Koninklijke Philips N.V., 5656 AG Eindhoven (NL)
(72) Inventor: STIGALL, Jeremy, 5656 AE Eindhoven (NL); SAROHA, Princeton, 5656 AE Eindhoven (NL)
(74) Representative: Philips Intellectual Property & Standards
(86) International application number: PCT/EP2017/057478
(87) International publication number: WO 2017/167841

(56) References cited:
- US-A1- 2011 301 458
- US-A1- 2013 338 498
- US-A1- 2014 221 842

## Description

### TECHNICAL FIELD

The present disclosure relates generally to imaging and mapping vascular pathways and surrounding tissue with photoacoustic and ultrasound modalities.

### BACKGROUND

Innovations in diagnosing and verifying the level of success of treatment of disease have migrated from external imaging processes to internal diagnostic processes. In particular, diagnostic equipment and processes have been developed for diagnosing vasculature blockages and other vasculature disease by means of ultra-miniature sensors placed upon the distal end of a flexible measurement apparatus such as a catheter, or a guide wire used for catheterization procedures. For example, known medical sensing techniques include angiography, intravascular ultrasound (IVUS), forward looking IVUS (FL-IVUS), fractional flow reserve (FFR) determination, a coronary flow reserve (CFR) determination, optical coherence tomography (OCT), trans-esophageal echocardiography, and image-guided therapy.

For example, intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. There are two general types of IVUS devices in use today: rotational and solid-state (also known as synthetic aperture phased array). For a typical rotational IVUS device, a single ultrasound transducer element is located at the tip of a flexible driveshaft that spins inside a plastic sheath inserted into the vessel of interest. In side-looking rotational devices, the transducer element is oriented such that the ultrasound beam propagates generally perpendicular to the longitudinal axis of the device. In forward-looking rotational devices, the transducer element is pitched towards the distal tip so that the ultrasound beam propagates more towards the tip (in some devices, being emitted parallel to the longitudinal centerline). The fluid-filled sheath protects the vessel tissue from the spinning transducer and driveshaft while permitting ultrasound signals to propagate from the transducer into the tissue and back. As the driveshaft rotates, the transducer is periodically excited with a high voltage pulse to emit a short burst of ultrasound. The same transducer then listens for the returning echoes reflected from various tissue structures. The IVUS medical sensing system assembles a two dimensional display of the tissue, vessel, heart structure, etc. from a sequence of pulse/acquisition cycles occurring during a single revolution of the transducer. In order to image a length of a vessel, the transducer element is drawn through the vessel as it spins.

In contrast, solid-state IVUS devices utilize a scanner assembly that includes an array of ultrasound transducers connected to a set of transducer controllers. In side-looking and some forward-looking IVUS devices, the transducers are distributed around the circumference of the device. In other forward-looking IVUS devices, the transducers are a linear array arranged at the distal tip and pitched so that the ultrasound beam propagates closer to parallel with the longitudinal centerline. The transducer controllers select transducer sets for transmitting an ultrasound pulse and for receiving the echo signal. By stepping through a sequence of transmit-receive sets, the solid-state IVUS system can synthesize the effect of a mechanically scanned transducer element but without moving parts. Since there is no rotating mechanical element, the transducer array can be placed in direct contact with the blood and vessel tissue with minimal risk of vessel trauma. Furthermore, because there is no rotating element, the interface is simplified. The solid-state scanner can be wired directly to the medical sensing system with a simple electrical cable and a standard detachable electrical connector. While the transducers of the scanner assembly do not spin, operation is similar to that of a rotational system in that, in order to image a length of a vessel, the scanner assembly is drawn through the vessel while stepping through the transmit-receive sets to produce a series of radial scans.

Rotational and solid-state state IVUS are merely some examples of imaging modalities that sample a narrow region of the environment and assemble a two- or three-dimensional image from the results. Other examples include optical coherence tomography (OCT), which has been used in conjunction with ultrasound systems. One of the key challenges using these modalities with in a vascular pathway is that they are limited in gathering data on anatomy beyond the vessel walls. Although OCT imaging may yield higher resolution than IVUS imaging, OCT has particularly limited penetration depth and may take more time to image a region of tissue.

Another recent biomedical imaging modality is photoacoustic imaging. Photoacoustic imaging devices deliver a short laser pulse into tissue and monitor the resulting acoustic output from the tissue. Due to varying optical absorption throughout the tissue, pulse energy from the laser pulse causes differential heating in the tissue. This heating and associated expansion leads to the creation of sound waves corresponding to the optical absorption of the tissue. These sound waves can be detected and an image of the tissue can be generated through analysis of the sound waves and associated vascular structures can be identified, as described in U.S. Patent Publication 2013/0046167 titled "SYSTEMS AND METHODS FOR IDENTIFYING VASCULAR BORDERS,". A document US 2014/221842 discloses a photoacoustic device capable of performing both intravascular photoacoustic "IVPA" imaging and intravascular ultrasound imaging. The device includes one or more optical fibers coupled to a transducer assembly that includes an photoacoustic transducer and optionally an ultrasound transducer. The one or more optical fibers and photoacoustic transducer are arranged such that the illumination field generated by the one or more optical fibers is co-aligned with the sensitive region of the photoacoustic transducer. Another document US 2013/338498 A1 discloses a method of imaging and treating a target tissue without the need to occlude or dilute luminal blood in a subject by a combination of intravascular ultrasound and photoacoustic imaging by irradiating the target tissue with electromagnetic radiation at a single wavelength.

Accordingly, for these reasons and others, the need exists for improved systems and techniques that allow for the mapping of vascular pathways and surrounding tissue.

### SUMMARY

Embodiments of the present disclosure provide systems that combine photoacoustic and IVUS imaging techniques. The systems may be used to image and/or map vascular pathways and surrounding tissue.

In one embodiment, a medical sensing system comprising a measurement apparatus according to claim 1 is provided.

The measurement apparatus comprises at least one ultrasound transducer configured to transmit ultrasound signals and receive ultrasound echo signals based on the transmitted ultrasound signals. The measurement apparatus further comprises at least one photoacoustic transducer configured to receive sound waves generated by the tissue as a result of interaction of the laser pulses with the tissue.

In some embodiments, one or more laser emitters are disposed outside the body of the patient. The region of interest may comprise the vascular pathway and a region of tissue surrounding the vascular pathway. The at least one photoacoustic transducer and the at least ultrasound transducer are configured to alternate in measuring sound waves and ultrasound echo signals, wherein during the time that the at least one photoacoustic transducer and the at least one ultrasound transducer is measuring sound waves or ultrasound echo signals, the other of the at least one photoacoustic transducer and the at least one ultrasound transducer does not measure sound waves or ultrasound echo signals. The processing engine may be further operable to control the operation of the at least one ultrasound and photoacoustic transducer, may be operable to activate the at least one ultrasound transducer to transmit ultrasound signals, and may be operable to activate the at least one photoacoustic transducer to receive at least one of the sound waves and the ultrasound echo signals.

In some embodiments, the at least one ultrasound and photoacoustic transducer is disposed circumferentially around a distal portion of the measurement apparatus. The at least one ultrasound and photoacoustic transducer may be coupled to a drive member that rotates the at least one ultrasound and photoacoustic transducer around a longitudinal axis of the measurement apparatus. The apparatus may comprise two or more laser emitters configured to emit laser pulses to tissue of the patient in the region of interest, and the two or more laser emitters may be configured to emit laser pulses simultaneously.

In some embodiments, at least one of the two or more laser emitters is configured to emit laser pulses at an oblique angle with respect to a longitudinal axis of the measurement apparatus. The one or more laser emitters may be disposed on an array outside the body of the patient. Furthermore, the array may have an arcuate shape.

In some embodiments, not part of the invention, a method of producing an image of a region of interest is provided, comprising: transmitting, with a laser source disposed outside a body of a patient, a focused laser pulse on tissue in a region of interest having at least one vascular pathway; receiving, with at least one photoacoustic sensor positioned within the vascular pathway of the region of interest, sound waves generated by the tissue in response to an interaction of the focused laser pulse with the tissue; transmitting, with at least one ultrasound transducer positioned within the vascular pathway of the region of interest, ultrasound signals toward the tissue in the region of interest; receiving, with the at least one ultrasound transducer positioned within the vascular pathway of the region of interest, ultrasound echo signals of the transmitted ultrasound signals; producing an image of the region of interest based on the received sound waves and the received ultrasound echo signals; and outputting the image of the region of interest to a display.

In some embodiments, not part of the invention, the method further comprises rotating at least one of the at least one photoacoustic sensor and the at least one ultrasound transducer within the vascular pathway about a longitudinal axis of an intravascular device to which the at least one photoacoustic sensor and/or the at least one ultrasound transducer is coupled. The method may also comprise positioning two or more laser sources outside the body of the patient around the region of interest. In some embodiments, the method further comprises positioning the two or more laser sources in an arcuate array outside the body of the patient around the region of interest. The two or more laser sources may transmit focused laser pulses simultaneously.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig 1A is a diagrammatic schematic view of a medical sensing system according to some embodiments of the present disclosure.
Fig 1B is a diagrammatic schematic view of a medical sensing system according to some embodiments of the present disclosure.
Fig 1C is a diagrammatic schematic view of a medical sensing system with an exemplary sensor array according to some embodiments of the present disclosure.
Fig 1D is a diagrammatic schematic view of a medical sensing system with another exemplary sensor array according to some embodiments of the present disclosure.
Fig. 2 is a diagrammatic, perspective view of a vascular pathway and surrounding tissue with an instrument positioned within the pathway and an external emitter according to an embodiment of the present disclosure.
Fig. 3 is a diagrammatic, perspective view of a vascular pathway and surrounding tissue with an instrument positioned within the pathway and two external emitters according to an embodiment of the present disclosure.
Fig. 4 is a diagrammatic, perspective view of a vascular pathway and surrounding tissue with an instrument positioned within the pathway and an external emitter array according to an embodiment of the present disclosure.
Fig. 5 is a flow diagram of a method for mapping a vascular pathway with photoacoustic and ultrasound modalities according to some embodiments of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the intravascular sensing system is described in terms of cardiovascular imaging, it is understood that it is not intended to be limited to this application. The system is equally well suited to any application requiring imaging within a lumen or cavity of a patient. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

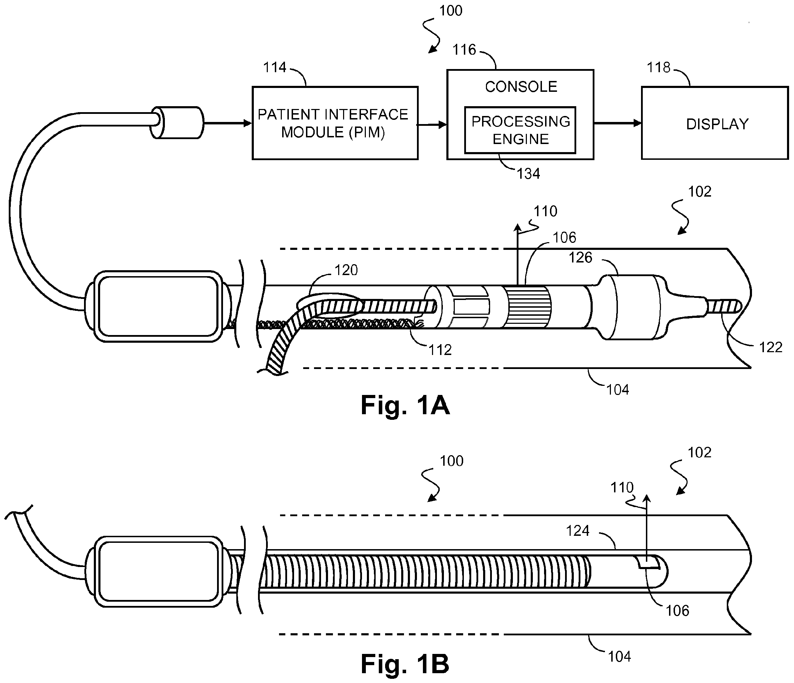

Fig. 1A is a diagrammatic schematic view of a medical sensing system 100 according to some embodiments of the present disclosure. The medical sensing system 100 may include a measurement apparatus 102 (such as a catheter, guide wire, or guide catheter). As used herein, "measurement apparatus" or "flexible measurement apparatus" includes at least any thin, long, flexible structure that can be inserted into the vasculature of a patient. While the illustrated embodiments of the "measurement apparatus" of the present disclosure have a cylindrical profile with a circular cross-sectional profile that defines an outer diameter of the flexible measurement apparatus 102, in other instances, all or a portion of the flexible measurement apparatus 102 may have other geometric cross-sectional profiles (e.g., oval, rectangular, square, elliptical, etc.) or non-geometric cross-sectional profiles. Flexible measurement apparatus 102 may include, for example, guide wires, catheters, and guide catheters. In that regard, a catheter may or may not include a lumen extending along all or a portion of its length for receiving and/or guiding other instruments. If the catheter includes a lumen, the lumen may be centered or offset with respect to the cross-sectional profile of the device.

The medical sensing system 100 may be utilized in a variety of applications and can be used to assess vessels and structures within a living body. To do so, the measurement apparatus 102 is advanced into a vessel 104. Vessel 104 represents fluid filled or surrounded structures, both natural and man-made, within a living body that may be imaged and can include for example, but without limitation, structures such as: organs including the liver, heart, kidneys, as well as valves within the blood or other systems of the body. In addition to imaging natural structures, the images may also include man-made structures such as, but without limitation, heart valves, stents, shunts, filters and other devices positioned within the body. The measurement apparatus 102 includes one or more sensors 106 disposed along the length of the apparatus 102 to collect diagnostic data regarding the vessel 104. In various embodiments, the one or more sensors 106 correspond to sensing modalities such as IVUS imaging, pressure, flow, OCT imaging, transesophageal echocardiography, temperature, other suitable modalities, and/or combinations thereof.

In the exemplary embodiment of Fig. 1A, the measurement apparatus 102 includes a solid-state IVUS device, and the sensors 106 include one or more IVUS ultrasound transducers and/or photoacoustic transducers and associated control. As used herein, a "photoacoustic transducer" includes at least a sensor configured to detect photoacoustic waves generated as a result of the interaction of optical pulses with tissue. In one embodiment, a photoacoustic transducer utilizes the same ultrasound detection mechanism as an IVUS ultrasound transducer. In some implementations, a single transducer can serve as both an IVUS transducer and a photoacoustic transducer. In another embodiment, a photoacoustic transducer uses a dedicated photoacoustic wave detection mechanism distinct from that of an IVUS ultrasound transducer. The system of Fig. 1A may include aspects of phased-array IVUS devices, systems, and methods associated with the Eagle Eye® Platinum catheter available from Volcano Corporation as well as those described in U.S. Patent No. 7,846,101 and/or U.S. Patent Application No. 14/812,792, filed July 29, 2015.

The sensors 106 may be arranged around the circumference of the measurement apparatus 102 and positioned to emit ultrasound energy radially 110 in order to obtain a cross-sectional representation of the vessel 104 and the surrounding anatomy. When the sensors 106 are positioned near the area to be imaged, the control circuitry selects one or more IVUS transducers to transmit an ultrasound pulse that is reflected by the vessel 104 and the surrounding structures. The control circuitry also selects one or more transducers to receive the ultrasound echo signal. By stepping through sequences of transmit-receive sets, the medical sensing system 100 system can synthesize the effect of a mechanically scanned transducer element without moving parts.

In one embodiment, the sensors 106 are disposed circumferentially around a distal portion of the measurement apparatus 102. In another embodiment, the sensors 106 are contained within the body of the measurement apparatus 102. In other embodiments, the sensors 106 are disposed radially across the measurement apparatus 102, on a movable drive member connected to the measurement apparatus 102, or on one or more planar arrays connected to the measurement apparatus 102.

In some embodiments, the processing engine 134, which may be included in the console 116, combines the imaging data acquired from both the IVUS and photoacoustic modalities into a single visualization. This use of both IVUS and photoacoustic modalities may provide a number of advantages over traditional systems using a single modality. First, the addition of photoacoustic sensors may allow for higher resolution mapping than traditional IVUS methods alone. Second, the combination of IVUS and photoacoustic modalities may allow for faster imaging speeds than OCT imaging or other methods. Third, the combination may allow for two-dimensional and/or three-dimensional imaging of the tissue surrounding vascular pathways. Fourth, the use of photoacoustic imaging may expand the diagnostic scope of an IVUS mapping procedure by including more of the surrounding tissue. In particular, the combined IVUS and photoacoustic mapping can allow for detection of certain types of cancers, tissue damage, and the mapping of multiple vascular pathways without sacrificing the dependability of ultrasound in detecting plaques, stenosis, and other forms of vascular diseases. Fifth, combining these two modalities may allow substantial costs savings because existing IVUS systems may be adapted to mapping systems using both modalities. Sixth, due to the interaction of optical pulses with tissue and the omni-directional emission of photoacoustic waves from the tissue, an optical pulse need not be emitted along the same axis as the transducer. This allows for more flexibility in carrying out combined photoacoustic and IVUS procedures, and may allow for precise mapping procedures even along deep or convoluted vascular pathways. Seventh, the mapping capabilities of the present disclosure may be integrated with some forms of laser therapy. For example, diagnosis of diseases in tissue may be accomplished using the optical emitter in diagnostic mode. After a diagnosis, the optical emitter can be switched to a treatment mode. In this regard, the map of the vasculature and surrounding tissue may be used to guide the application of the treatment. After the optical treatment is finished, the optical emitter can be switched back to diagnostic mode to confirm treatment of the diseased portion of tissue.

Sensor data may be transmitted via a cable 112 to a Patient Interface Module (PIM) 114 and to console 116, as well as to the processing engine 134 which may be disposed within the console 116. Data from the one or more sensors 106 may be received by a processing engine 134 of the console 116. In other embodiments, the processing engine 134 is physically separated from the measurement apparatus 102 but in communication with the measurement apparatus (e.g., via wireless communications). In some embodiments, the processing engine 134 is configured to control the sensors 106. Precise timing of the transmission and reception of signals may be used to map vascular pathways 104 in procedures using both IVUS and photoacoustic modalities. In particular, some procedures may involve the activation of sensors 106 to alternately transmit and receive signals. In systems using one or more IVUS transducers that are configured to receive both photoacoustic and ultrasound signals, the processing engine 134 may be configured to control the state (e.g., send/receive) of one or more transducers during the mapping of the vascular pathway and surrounding tissue.

Moreover, in some embodiments, the processing engine 134, PIM 114, and console 116 are collocated and/or part of the same system, unit, chassis, or module. Together the processing engine 134, PIM 114, and/or console 116 assemble, process, and render the sensor data for display as an image on a display 118. For example, in various embodiments, the processing engine 134, PIM 114, and/or the console 116 generates control signals to configure the sensor 106, generates signals to activate the sensor 106, performs amplification, filtering, and/or aggregating of sensor data, and formats the sensor data as an image for display. The allocation of these tasks and others can be distributed in various ways between the processing engine 134, PIM 114, and the console 116.

In addition to various sensors 106, the measurement apparatus 102 may include a guide wire exit port 120 as shown in Fig. 1A. The guide wire exit port 120 allows a guide wire 122 to be inserted towards the distal end in order to direct the member 102 through a vascular structure (*i.e.,* the vessel) 104. Accordingly, in some instances the measurement apparatus 102 is a rapid-exchange catheter. Additionally or in the alternative, the measurement apparatus 102 can be advanced through the vessel 104 inside a guide catheter 124. In an embodiment, the measurement apparatus 102 includes an inflatable balloon portion 126 near the distal tip. The balloon portion 126 is open to a lumen that travels along the length of the IVUS device and ends in an inflation port (not shown). The balloon 126 may be selectively inflated and deflated via the inflation port. In other embodiments, the measurement apparatus 102 does not include balloon portion 126.

Fig. 1B is a schematic view of a system that includes an alternative measurement apparatus 102 according to some embodiments of the present disclosure. The measurement apparatus 102 of Fig. 1B is typical of a rotational device such as a rotational IVUS ultrasound system and the one or more sensors 106 include one or more IVUS transducers arranged to emit ultrasound energy in a radial direction 110, as well as one or more photoacoustic transducers. Again, a single transducer may serve as both an IVUS transducer and a photoacoustic transducer. In such an embodiment, the one or more sensors 106 may be mechanically rotated around a longitudinal axis of the measurement apparatus 102 to obtain a cross-sectional representation of the vessel 104. The system of Fig. 1B may include aspects of rotational IVUS devices, systems, and methods associated with the Revolution® catheter available from Volcano Corporation as well as those described in U.S. Patent Nos. 5,243,988, 5,546,948, and 8,104,479 and/or U.S. Patent Application No. 14/837,829, filed August 27, 2015.

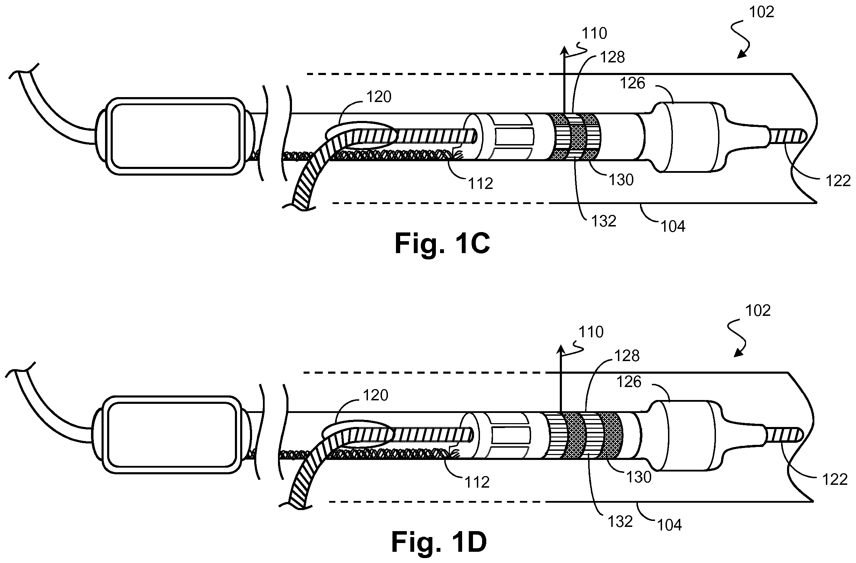

Fig. 1C and 1D show alternative sensor arrays 128 that may be used in conjunction with the measurement apparatus 102 according to some embodiments of the present disclosure. In particular, the sensor array 128 may include one or more sensors 106 and emitters including IVUS transducers, IVUS emitters, photoacoustic transducers, and optical emitters. In Fig. 1C, the sensor array 128 is disposed around the circumference of the measurement apparatus 102. Sensors 106 of two more different types are placed in the sensor array 128. In particular, sensors of a first type 130 are placed in the sensor array 128 with sensors of a second type 132. In the example of Fig. 1C, the sensors of the first and second types 130,132 are disposed on the array 128 in an alternating manner. In some embodiments, sensors of the first and second types 130, 132 are disposed on the array 128 in a checkerboard configuration such that individual sensors of the first type 130 are not adjacent to each other. Additionally, sensors of the first and second types 130, 132 may take up roughly equal proportions of the area of the array 128. Although they appear as square or rectangular in the example of Fig. 1C, sensors of the first and second types 130, 132 may have circular, elliptical, polygonal, or other shapes. Sensors of the first and second types 130, 132 may be spaced across the measurement apparatus 120 or they may be placed flush against each other.

In the example of Fig. 1D, a sensor array 128 is shown with sensors of two or more different types 130, 132 disposed in alternating rows. These rows may be disposed axially and may extend part way or completely around the measurement apparatus 102. In some embodiments, rows of sensors placed in a staggered formation and the ends of individual rows are not flush. In some embodiments, rows of sensors are placed adjacent to each other with no space in between. Alternatively, rows of sensors are spaced across the measurement apparatus 102 with space therebetween. In some cases, 2, 3, 4, or 5 rows of alternating sensors are disposed on the measurement apparatus 102. As discussed above, the array 128 may be configured to rotate around an axis of the measurement apparatus 102.

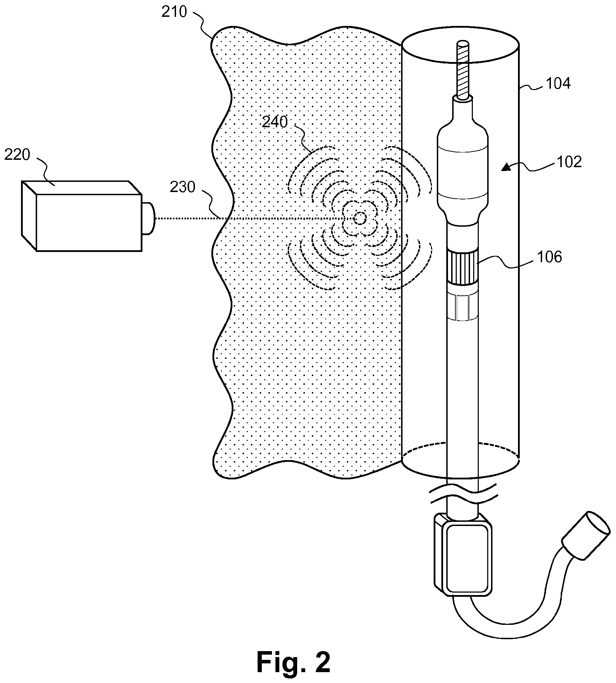

Fig. 2 is a diagrammatic, perspective view of a vascular pathway 104 and surrounding tissue 210 with a measurement apparatus 102 such as that depicted in Figs. 1A-1D disposed within the vascular pathway 104. An optical emitter 220 is also shown emitting an optical pulse 230 toward an area of interest within the tissue. In some embodiments, the area of interest includes part of a vascular pathway 104 as well as adjacent tissue. In some embodiments, the optical emitter 220 is a laser source that emits short laser pulses toward the area of interest. These laser pulses interact with the tissue 210, generating a series of photoacoustic waves 240 that propagate through the tissue 210 as well as through the vascular pathway 104. The photoacoustic waves 240 are received by the sensors 106 connected to the measurement apparatus 102. The sensors 106 may also image and/or map the vascular pathway 104 independently of the photoacoustic waves 240, by transmitting ultrasound signals toward the vessel walls and receiving the corresponding reflected ultrasound echoes.

An operator may move the measurement apparatus 102 through the vascular pathway 104 to image and/or map the vascular pathways 104. In some cases, the optical emitter 220 is configured to emit optical pulses 230 toward the sensors 106 of the measurement apparatus. Accordingly, the optical emitter 220 may be moved at a similar speed and direction as the measurement apparatus 102.

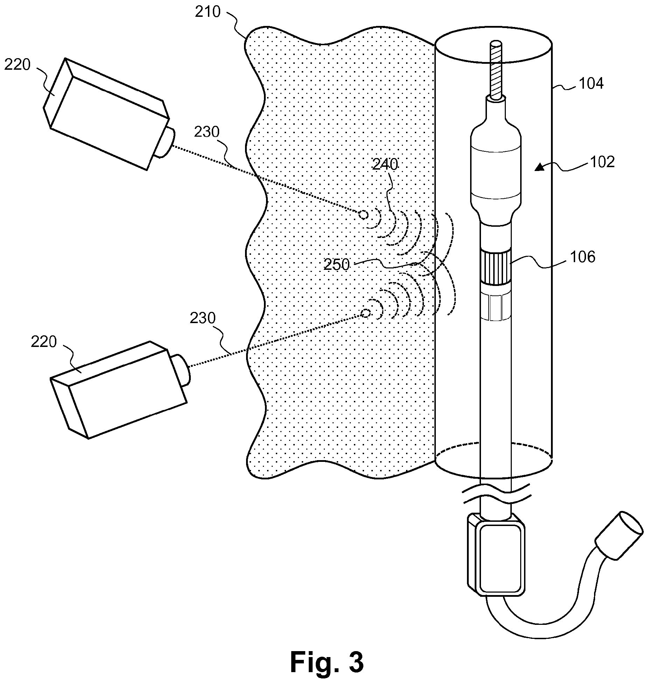

Fig. 3 shows a mapping system using two optical emitters 220 that emit optical pulses toward the sensors 106 of the measurement apparatus 102. In some embodiments, at least one of the optical emitters 220 is configured to emit optical pulses at an oblique angle with respect to a longitudinal axis of the measurement apparatus 102. The use of two or more optical emitters 220 may allow more accurate mapping of the area of interest. In particular, the emission of optical pulses 230 from different sources may generate interference patterns 250 between the photoacoustic waves 240 emanating from the tissue 210. These interference patterns may be analyzed by the processing engine 134 to produce additional data points for use in tissue mapping. In some embodiments, the optical emitters emit optical pulses into the tissue 210 in different patterns. In some embodiments, three, four, five, or six optical emitters are used together to map a region of interest.

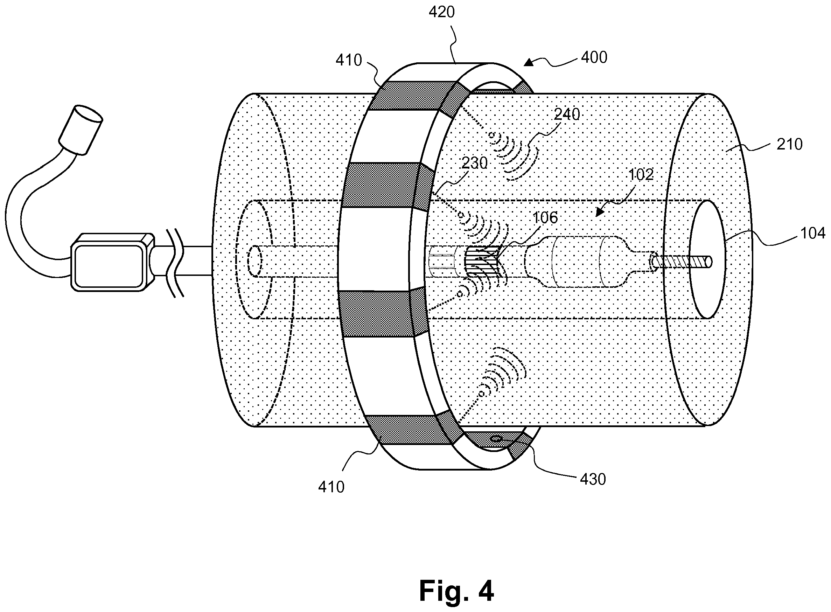

Fig. 4 shows a measurement apparatus 102 disposed within a vascular pathway 104 and surrounding tissue 210. An emitter array 400 comprising a plurality of optical emitters 410 is disposed around the tissue 210. The example of Fig. 4 shows an emitter array 400 with an arcuate ring shape. The emitter array 400 may also have a hexagon, octagon, or other polygonal shape, *etc.* This shape may allow for placement of the emitter array 400 around an extremity of a patient, such as around an arm or leg. In other embodiments, the emitter array 400 has one or more flat surfaces that allow at least a portion of the emitter array 400 to be placed parallel to a flat portion of tissue 210, such as the abdomen of a patient. In some embodiments, the optical emitters 410 simultaneously emit optical pulses 240 into the tissue 210. In other embodiments, the optical emitters 410 alternatively emit optical pulses 240 into the tissue 210. For example, the emitters 420 may emit pulses consecutively around the circumference of the emitter array 400. The use of such an emitter array 400 may allow for a faster photo acoustic imaging rates by providing imaging and/or mapping of a large swath of tissue without rotation of an optical emitter itself, which may be particularly useful for imaging and/or mapping extremities. Additionally, the use of an emitter array 400 may allow for the simultaneous production of photoacoustic waves in different areas of tissue 210 while avoiding destructive interference between waves.

In one embodiment, a plurality of transducers, each corresponding to an optical emitter 410 of the emitter array 400, are included in the sensors. In this case, the emitter array 400 and measurement apparatus 102 are moved at a similar speed to ensure that each transducer receives photoacoustic signals generated as a result of the corresponding emitter 410. In an alternative embodiment, the individual emitters 410 do not correspond to individual sensors. In this case, the emitter array 400 can contain a different number of emitters 410 than the number of sensors. Each sensor instead receives the signals that are directed toward its respective location regardless of the emitter 410 that caused the generation of the signals.

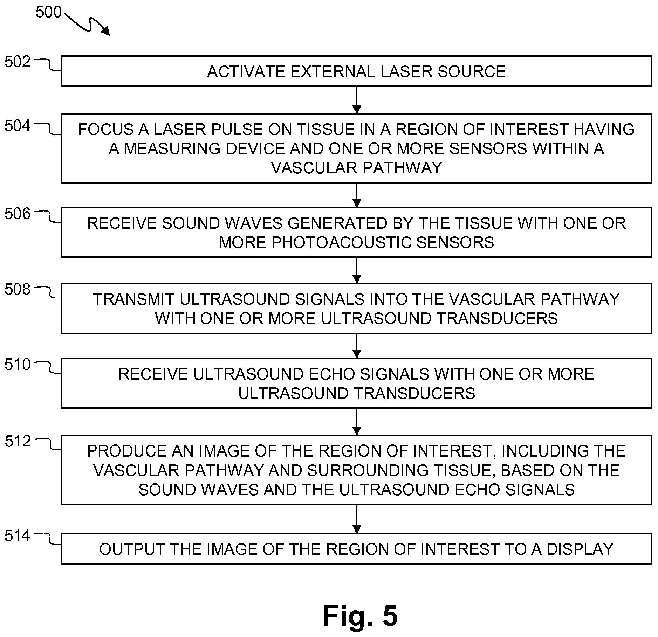

Fig. 5 is a flow chart showing a method 500 of mapping an area of interest using both photoacoustic and IVUS modalities. It is understood that additional steps can be provided before, during, and after the steps of method 500, and that some of the steps described can be replaced or eliminated for other embodiments of the method. In particular, steps 504, 506, 508, and 510 may be performed simultaneously or in various sequences as discussed below.

At step 502, the method 500 can include activating an external laser source. This laser source may be the optical emitter 220 of Fig. 3. In some cases, the external laser source is activated by a communication system 250 by means of an electronic or optical signal. This signal may be sent wirelessly, and the external laser source may be equipped with a wireless signal receiver.

At step 504, the method 500 can include focusing a laser pulse on tissue in a region of interest having a measuring device and one or more sensors within a vascular pathway. In some embodiments, the region of interest includes a portion of tissue including a portion of at least one vascular pathway 104, and the measuring device may be disposed within the vascular pathway 104. The region of interest may be chosen based on a suspected or diagnosed problem in the tissue, or based on the proximity of a region of tissue to problems within a vascular pathway 104. In other embodiments, the region of interest is part of a more general mapping plan. For example, a mapping plan for a section of a vascular pathway 104 may involve the mapping of tissue surrounding the vascular pathway 104 along its length. The one or more photoacoustic sensors may be disposed on or within the measurement apparatus. In some cases, the one or more photoacoustic signals are disposed circumferentially around the measurement apparatus. The interaction of the emitted laser pulse and tissue in the region of interest creates a number of photoacoustic waves 240 that emanate from the tissue.

In some embodiments, the measuring device is the measurement apparatus 102 depicted in Figs. 1A, 1B, 2A-2E, 3, and 4. In some embodiments, sensors are those depicted in Figs. 1A, 1B, and 2-4, and can be included in the sensor array 128 depicted in Figs. 2A-2E. The sensors can include IVUS transducers, IVUS emitters, OCT transducers, photoacoustic transceivers, and optical emitters. The sensors can be arranged in any of the examples depicted in Figs. 2A-2E. In some embodiments, the sensors do not rotate as they travel through the vascular pathway 104. In other embodiments, the sensors are included in a rotational array disposed on a revolving portion of the measurement device. In some embodiments, the sensors are disposed circumferentially around the measurement device.

At step 506, the method 500 can include receiving sound waves generated by the interaction of the laser pulse and tissue with one or more photoacoustic sensors. In some cases, the one or more photoacoustic sensors can function as a traditional IVUS imaging element. In other cases, the one or more photoacoustic sensors are dedicated to receiving photoacoustic waves without IVUS functionality. In some embodiments, the one or more photoacoustic sensors are controlled by a communication system 250 like that depicted in Figs. 3 and 4. In another embodiment, a processing engine 130 or a PIM 114 may control sensors on a sensor array 128. Signals may be sent from processing engine 130 or the PIM 114 to the sensors, including the one or more photoacoustic sensor, via connector 234, causing the one or more photoacoustic sensor to receive diagnostic information such as sound waves or ultrasound signals.

At step 508, the method 500 can include transmitting ultrasound signals into the vascular pathway 104 with one or more ultrasound transducers. The ultrasound signals may be transmitted toward the walls of the vascular pathway 104 and may be deflected off the walls of the vascular pathway 104 and propagate through the vascular pathway 104 as ultrasound echo signals. In some embodiments, the one or more ultrasound transducers are disposed on a solid-state array positioned within the vascular pathway. The one or more ultrasound transducers may be coupled to a drive member that rotates around a longitudinal axis of the measurement apparatus. The transmitted ultrasound signals are deflected off the walls of the vascular pathway 104 and propagate through the vascular pathway 104 as ultrasound echo signals.

At step 510, the method 500 can include receiving the ultrasound echo signals with the one or more ultrasound transducers. In some embodiments, the one or more ultrasound transducers may be operable to receive sound waves as well as ultrasound signals. The one or more ultrasound transducers of step 508 and the one or more ultrasound transducers of step 510 may be combined in a single element, or the transducer elements may be separate.

Steps 504, 506, 508, and 510 may be coordinated in the method 500 and occur in various orders based on the desired outcome of a medical procedure. For example, transmission of ultrasound signals and reception of ultrasound echo signals can occur at regular intervals throughout the method 500, while reception of photoacoustic waves may occur sporadically. This may be the case in a medical procedure to map a vascular pathway 104 and spot-check trouble areas of tissue surrounding sections of the vascular pathway 104. Alternatively, steps 504, 506, 508, and 510 are performed successively. For example, steps 506, 508, and 510 may be performed individually before proceeding to the next step to avoid signal noise and allow for adequate signal processing when method 500 is used in a system where a photoacoustic sensor and ultrasound transducer are each included in a transducer array. Furthermore, the steps of method 500 may be interleaved in various orders.

At step 512, the method 500 can include producing an image of the region of interest, including the vascular pathway 104 and surrounding tissue, based on the sound waves and the ultrasound echo signals. In some embodiments, a processing engine (such as the processing engine 130 of Fig. 1A) in communication with the sensors produces the image of the region of interest. This image can include both two-dimensional and three-dimensional images based on the received sensor data. In some cases, the image includes a number of two-dimensional cross sections of the vascular pathway 104 and surrounding tissue.

At step 514, the method 500 can include outputting the image of the region of interest to a display 118. This display 118 can include a computer monitor, a screen on a patient interface module (PIM) 114 or console 116, or other suitable device for receiving and displaying images.

In an exemplary embodiment within the scope of the present disclosure, the method 500 repeats after step 514, such that method flow goes back to step 504 and begins again. Iteration of the method 500 may be utilized to map a vascular pathway and surrounding tissue.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A medical sensing system comprising:
one or more laser emitters (220, 420) configured to emit laser pulses (230) to tissue (210) of a patient in a region of interest;
a measurement apparatus (102) configured to be placed within a vascular pathway (104) in the region of interest, wherein the measurement apparatus (102) comprises at least one ultrasound transducer (106) configured to transmit ultrasound signals and receive ultrasound signals based on the transmitted ultrasound signals; at least one photoacoustic transducer (106) configured to receive sound waves generated by the tissue as a result of interaction of the laser pulses with the tissue; wherein the at least one photoacoustic transducer and the at least one ultrasound transducer are configured to alternate in measuring sound waves and ultrasound echo signals;
wherein the one or more laser emitters (220, 420) are disposed outside the body of the patient and configured to emit the optical pulses (230) toward the at least one photoacoustic transducer (106);
a processing engine (134) in communication with the measurement apparatus (102), the processing engine (134) operable to produce an image of the region of interest based on the received sound waves (240) and the received ultrasound echo signals; and
a display (118) in communication with the processing engine (134), the display (118) configured to visually display the image of the region of interest.

2. The medical sensing system of claim 1, wherein the region of interest comprises the vascular pathway (104) and a region of tissue surrounding the vascular pathway (104).

3. The medical sensing system of claim 1, wherein the processing engine (134) is further operable to control the operation of the at least one ultrasound or photoacoustic transducer (106).

4. The medical sensing system of claim 3, wherein the processing engine (134) is further operable to activate the at least one ultrasound transducer (106) to transmit ultrasound signals.

5. The medical sensing system of claim 1, wherein the at least one ultrasound and photoacoustic transducer (106) is disposed circumferentially around a distal portion of the measurement apparatus (102).

6. The medical sensing system of claim 1, wherein the at least one ultrasound and photoacoustic transducer (106) is coupled to a drive member configured to rotate the at least one ultrasound and photoacoustic transducer (106) around a longitudinal axis of the measurement apparatus (102).

7. The medical sensing system of claim 1, comprising two or more laser emitters (220, 420) configured to emit laser pulses to tissue of the patient in the region of interest.

8. The medical sensing system of claim 7, wherein the two or more laser emitters (220, 420) are configured to emit laser pulses simultaneously; or
wherein at least one of the two or more laser emitters (220, 420) is configured to emit laser pulses at an oblique angle with respect to a longitudinal axis of the measurement apparatus (102).

9. The medical sensing system of claim 1, wherein the one or more laser emitters (220, 420) is disposed on an array (400) outside the body of the patient; and
wherein, preferably, the array has an arcuate shape.

## Patentansprüche

1. Medizinisches Erfassungssystem, umfassend:
einen oder mehrere Laserstrahler (220, 420), die konfiguriert sind, um Laserpulse (230) an Gewebe (210) eines Patienten in einem interessierenden Bereich zu emittieren;
eine Messvorrichtung (102), die konfiguriert ist, um in einer Gefäßbahn (104) in dem interessierenden Bereich angeordnet zu sein, wobei die Messvorrichtung (102) mindestens einen Ultraschallwandler (106) umfasst, der konfiguriert ist, um Ultraschallsignale zu senden und Ultraschallsignale basierend auf den übertragenen Ultraschallsignalen zu empfangen; mindestens einen photoakustischen Wandler (106), der konfiguriert ist, um Schallwellen zu empfangen, die vom Gewebe als Ergebnis der Wechselwirkung der Laserpulse mit dem Gewebe erzeugt werden; wobei der mindestens eine photoakustische Wandler und der mindestens eine Ultraschallwandler konfiguriert sind, um sich beim Messen von Schallwellen und Ultraschallechosignalen abzuwechseln;
wobei der eine oder die mehreren Laserstrahler (220, 420) außerhalb des Körpers des Patienten angeordnet und konfiguriert sind, um die optischen Impulse (230) in Richtung des mindestens einen photoakustischen Wandlers (106) zu emittieren;
eine Verarbeitungsmaschine (134) in Verbindung mit der Messvorrichtung (102), wobei die Verarbeitungsmaschine (134) betreibbar ist, um ein Bild des interessierenden Bereichs basierend auf den empfangenen Schallwellen (240) und den empfangenen Ultraschallechosignalen zu erzeugen; und
eine Anzeige (118) in Kommunikation mit der Verarbeitungsmaschine (134), wobei die Anzeige (118) konfiguriert ist, um das Bild des interessierenden Bereichs visuell anzuzeigen.

2. Medizinisches Erfassungssystem nach Anspruch 1, wobei der interessierende Bereich die Gefäßbahn (104) und einen die Gefäßbahn (104) umgebenden Gewebebereich umfasst.

3. Medizinisches Erfassungssystem nach Anspruch 1, wobei die Verarbeitungsmaschine (134) ferner betreibbar ist, um den Betrieb des mindestens einen Ultraschall- oder photoakustischen Wandlers (106) zu steuern.

4. Medizinisches Erfassungssystem nach Anspruch 3, wobei die Verarbeitungsmaschine (134) ferner betreibbar ist, um den mindestens einen Ultraschallwandler (106) zum Übertragen von Ultraschallsignalen zu aktivieren.

5. Medizinisches Erfassungssystem nach Anspruch 1, wobei der mindestens eine Ultraschall- und photoakustische Wandler (106) in Umfangsrichtung um einen distalen Abschnitt der Messvorrichtung (102) angeordnet ist.

6. Medizinisches Erfassungssystem nach Anspruch 1, wobei der mindestens eine Ultraschall- und photoakustische Wandler (106) mit einem Antriebselement gekoppelt ist, das konfiguriert ist, um den mindestens einen Ultraschall- und photoakustischen Wandler (106) um eine Längsachse der Messvorrichtung zu drehen (102).

7. Medizinisches Erfassungssystem nach Anspruch 1, umfassend zwei oder mehr Laserstrahler (220, 420), die konfiguriert sind, um Laserpulse an das Gewebe des Patienten in dem interessierenden Bereich zu emittieren.

8. Medizinisches Erfassungssystem nach Anspruch 7, wobei die zwei oder mehr Laserstrahler (220, 420) konfiguriert sind, um Laserpulse gleichzeitig zu emittieren; oder
wobei mindestens einer der zwei oder mehr Laserstrahler (220, 420) konfiguriert ist, um Laserpulse in einem schrägen Winkel in Bezug auf eine Längsachse der Messvorrichtung (102) zu emittieren.

9. Medizinisches Erfassungssystem nach Anspruch 1, wobei der eine oder die mehreren Laserstrahler (220, 420) auf einer Anordnung (400) außerhalb des Körpers des Patienten angeordnet sind; und
wobei die Anordnung vorzugsweise eine bogenförmige Form aufweist.

## Revendications

1. Système de détection médical comprenant:
un ou plusieurs émetteurs laser (220, 420) configurés pour émettre des impulsions laser (230) vers le tissu (210) d'un patient dans une région d'intérêt;
un appareil de mesure (102) configuré pour être placé dans une voie vasculaire (104) dans la région d'intérêt, où l'appareil de mesure (102) comprend au moins un transducteur ultrasonore (106) configuré pour transmettre des signaux ultrasonores et recevoir des signaux ultrasonores basés sur les signaux ultrasonores transmis; au moins un transducteur photoacoustique (106) configuré pour recevoir des ondes sonores générées par le tissu à la suite de l'interaction des impulsions laser avec le tissu; où l'au moins un transducteur photoacoustique et l'au moins un transducteur ultrasonore sont configurés pour alterner dans la mesure des ondes sonores et des signaux d'écho ultrasonores;
où l'un ou les plusieurs émetteurs laser (220, 420) sont disposés à l'extérieur du corps du patient et configurés pour émettre les impulsions optiques (230) vers l'au moins un transducteur photoacoustique (106) ;
un moteur de traitement (134) en communication avec l'appareil de mesure (102), le moteur de traitement (134) étant actionnable pour produire une image de la région d'intérêt sur la base des ondes sonores reçues (240) et des signaux d'écho ultrasonores reçus; et
un afficheur (118) en communication avec le moteur de traitement (134), l'afficheur (118) étant configuré pour afficher visuellement l'image de la région d'intérêt.

2. Système de détection médical selon la revendication 1, dans lequel la région d'intérêt comprend la voie vasculaire (104) et une région de tissu entourant la voie vasculaire (104).

3. Système de détection médical selon la revendication 1, dans lequel le moteur de traitement (134) est actionnable en outre pour commander le fonctionnement de l'au moins un transducteur ultrasonore ou photoacoustique (106).

4. Système de détection médical selon la revendication 3, dans lequel le moteur de traitement (134) est actionnable en outre pour activer l'au moins un transducteur ultrasonore (106) pour transmettre des signaux ultrasonores.

5. Système de détection médical selon la revendication 1, dans lequel l'au moins un transducteur ultrasonore et photoacoustique (106) est disposé circonférentiellement autour d'une partie distale de l'appareil de mesure (102).

6. Système de détection médical selon la revendication 1, dans lequel l'au moins un transducteur ultrasonore et photoacoustique (106) est couplé à un élément d'entraînement configuré pour faire tourner l'au moins un transducteur ultrasonore et photoacoustique (106) autour d'un axe longitudinal de l'appareil de mesure (102).

7. Système de détection médical selon la revendication 1, comprenant deux ou plusieurs émetteurs laser (220, 420) configurés pour émettre des impulsions laser vers le tissu du patient dans la région d'intérêt.

8. Système de détection médical selon la revendication 7, dans lequel les deux ou plusieurs émetteurs laser (220, 420) sont configurés pour émettre des impulsions laser simultanément; ou
où au moins l'un des deux ou plusieurs émetteurs laser (220, 420) est configuré pour émettre des impulsions laser à un angle oblique par rapport à un axe longitudinal de l'appareil de mesure (102).

9. Système de détection médical selon la revendication 1, dans lequel l'un ou les plusieurs émetteurs laser (220, 420) sont disposés sur un réseau (400) à l'extérieur du corps du patient; et
dans lequel, de préférence, le réseau a une forme arquée.
